# EUROPEAN PATENT APPLICATION

(11) **EP 4 445 855 A1**
(43) Date of publication of application: **16.10.2024**
(21) Application number: 23167745.1
(22) Date of filing: 13.04.2023
(51) Int. Cl.: A61B 18/14, A61N 1/04

(54) **METHOD AND CATHETER FOR RENAL DENERVATION**

(71) Applicant: BIOTRONIK SE & Co. KG, 12359 Berlin (DE)
(72) Inventor: Panescu, Dorin, San Jose, 95136 (US); Wildau, Hans-Jürgen, 10245 Berlin (DE); Bode, Sven, 10829 Berlin (DE); Hata, Cary, Irvine, 92620 (US); de la Rama, Alan, Cerritos, 90703 (US); Holzinger, Steffen, 12437 Berlin (DE)
(74) Representative: Biotronik Corporate Services SE

(57) **Abstract**

The invention relates to a catheter (1) and to a method for renal denervation. The catheter comprises a plurality of electrodes (9) arranged at a distal portion (13) of the catheter (1) and a PFA pulse generator (29) configured for generating electric PFA pulses. Furthermore, the catheter (1) is configured for implementing the method for renal denervation comprising applying electric PFA pulses to a vascular wall of a renal vessel at a position adjacent to nerves of a patient's sympathetic nervous system. Therein, the PFA pulses are applied via at least two electrodes (9) of the catheter (1) arranged within the renal vessel. The PFA pulses are configured such as to induce electroporation in nerve cells of the patient's sympathetic nervous system.

## Description

The present invention relates to a method and a catheter for renal denervation.

A patient's sympathetic nervous system may generally influence a cardiac activity of the patient's heart. Portions of the sympathetic nervous system extend in close proximity to a renal artery. Renal denervation is known as a therapeutic approach for affecting nerves of the sympathetic nervous system such as to specifically influence characteristics of the patient's heart activity.

Renal denervation using radiofrequency (RF) catheter ablation is known to eliminate at least portions of a patient's renal sympathetic nerve and to possibly lower blood pressure in patients with resistant hypertension. The sympathetic nervous system is a key component in the development of cardiovascular disease, and especially in hypertension and heart failure. The kidneys are richly innervated with sympathetic nerve fibers, which play a pivotal role in hypertension by stimulating renin secretion and tubular sodium reabsorption and by reducing urinary sodium excretion. While surgical sympathectomy fell out of favor due to significant perioperative morbidity and adverse effects, introduction of a minimally invasive catheter-based renal sympathetic denervation (RDN) facilitated clinical application of sympathetic denervation and promoted further research.

According to the basic principle of RF ablation (RFA), heat generated by the RF is applied to sympathetic nerve fibres. Thereby, the nerve fibres may be destroyed. Therefore, typically a catheter comprising at least two electrodes is introduced into the renal artery. RF energy is applied to the electrodes, thereby generating heat to destroy the nerve fibres close to the artery.

However, since the renal artery wall is between the electrodes and the nerve fibres, the artery wall may generally be affected by the applied heat as well. Accordingly, a problem of the RF ablation is applying enough thermal energy to destroy the nerve fibres and at the same time creating as little damage at the artery wall as possible.

Additionally, an effectiveness of RFA RDN may also depend on a thermal propagation of heat from the tissue surface in proximity to the electrodes at the RF catheter to the nerves behind the arterial wall. Typically, a renal artery blood flow is relatively high. Therefore, a significant part of the RF energy may be lost to the convective cooling provided by the arterial blood. As a result, insufficient heat may reach out to nerve fibers to cause permanent necrosis.

Accordingly, there may be a need for an alternative approach for addressing at least some of the above deficiencies of conventional renal denervation. Particularly, there may be a need for a method for renal denervation and for a catheter specifically configured for such renal denervation, wherein nerves in a patient's sympathetic nervous system located close to the renal artery may be modified or affected in a way such that, on the one hand, the nerves may be permanently and reliably deactivated while, on the other hand, negative side effects such as damages to neighbouring tissue of for example the renal artery wall may be minimised. Furthermore, there may be a need for an approach enabling renal denervation with consistent efficacy and/or with little or no dependence to an arterial blood flow.

Such need may be fulfilled by the subject matter of one of the independent claims. Advantageous embodiments are defined in the dependent claims, described in the present specification and visualised in the associated figures.

According to a first aspect of the present invention, a method for renal denervation is described. The method comprises applying electric pulse field ablation (PFA) pulses to a vascular wall of a renal vessel at a position adjacent to nerves of a patient's sympathetic nervous system. Therein, the PFA pulses are applied via at least two electrodes of a catheter arranged within the renal vessel. Furthermore, the PFA pulses are configured such as to induce electroporation in nerve cells of the patient's sympathetic nervous system

According to a second aspect, a catheter for renal denervation is described. The catheter comprises a plurality of electrodes arranged at a distal end of the catheter and wherein the catheter is configured for connecting to a PFA pulse generator configured for generating electric PFA pulses. The catheter is configured for implementing the method for renal denervation according to embodiments of the first aspect of the invention.

According to a third aspect, a system for renal denervation is described. The system is configured for implementing the method for renal denervation according to embodiments of the first aspect of the invention. Furthermore, the system comprises the catheter according to the second aspect of the invention and a PFA pulse generator configured for generating electrical PFA pulses according to the first aspect of the invention.

Ideas underlying embodiments of the present invention may be interpreted as being based, inter alia, on the following observations and recognitions.

Briefly summarised and without limiting the scope of the invention, a basic idea underlying embodiments of the invention and associated possible advantages will be roughly described as follows:
The above-mentioned needs may be fulfilled by applying pulse field ablation at the renal artery. Therein, ablation energy is provided in a form of short electric high voltage DC pulses, thereby locally generating high electric fields, typically in the range of hundreds to thousands of Volts/centimeter (V/cm) or more. It has been observed that such high electric fields may result in electroporation at membranes of living cells, eventually resulting in the cells being deactivated or resulting in cell death. PFA has been developed inter alia for ablating myocardial tissue. It was generally assumed that an electroporation threshold for nerve cells is usually very high. Consequently, while it was observed that PFA could be effectively applied for ablating myocardial tissue, it was not considered to apply high voltage PFA pulses in the renal artery to destroy the nerve fibres close to or attached to the artery wall.

However, it has now surprisingly been found that a reliable damage at nerve cells at the renal artery due to irreversible electroporation could be achieved by applying DC PFA voltage pulses upon suitably setting pulse application parameters. For example, surprisingly positive results have been observed upon setting the PFA voltage pulses to above specific high voltage threshold values of for example 4 kV. Moreover, it was observed that using pulse widths in a range of nanoseconds (ns) to microseconds (µs) may render the electroporation mechanism less tissue selective, thereby possibly allowing to circumvent the high ablation threshold of nerves. Furthermore, in such regime of short pulse durations the cell death mechanism appears to also involve permeabilization of a cell nucleus and organelles, thereby possibly allowing an ablation of nerve cells without destroying or causing stenosis in the renal artery.

Subsequently, possible features of embodiments of the inventive method, catheter and system for renal denervation and associated possible advantages will be described in more detail.

The approach described herein relies on the surprising finding that pulse field ablation, which was originally developed for ablating myocardial tissue in cardiac surgery, may be advantageously adopted for renal denervation. Thereby, an improved method and catheter for treating for example cardiac deficiencies such as resistant hypertension is made available for overcoming problems occurring in conventional renal denervation methods such as radiofrequency ablation.

PFA energy may generate irreversible electroporation of cell membranes and destroy tissue. Specifically, it is assumed that, upon application of PFA energy, strong electric fields may disrupt cell membranes by generating pores in the cell membrane, which subsequently destroys the cell membrane and the cell. While a precise mechanism of this electrically-driven pore generation, i.e. electroporation, is not yet understood in detail, it is assumed that an application of relatively large electric fields may generate instabilities e.g. in the phospholipid bilayers in cell membranes, as well as mitochondria. This may cause an occurrence of a distribution of local gaps or pores in the membrane. If the applied electric field at the membrane exceeds a threshold value, typically dependent on cell size and cell type, the electroporation is irreversible and the pores remain open, permitting exchange of material across the membrane and leading to apoptosis or cell death. Subsequently, the surrounding tissue may generally heal in a natural process.

Generally, an efficacy upon inducing electroporation may depend on various characteristics of the electric PFA pulses applied to treated cells as well as characteristics of the treated cells. It was now found that, if the characteristics of applied PFA pulses are selected correctly, even nerve cells of a patient's sympathetic nervous system may be ablated with PFA pulses, thereby overcoming the long felt prejudice by presenting the options for renal denervation presented herein.

According to an embodiment, the PFA pulses are applied by generating a voltage of at least 4 kV between the two electrodes. By setting a maximum voltage during a PFA pulse to a value above a predefined lower limit of 4 kV, effective electroporation may be induced even in nerve cells, such nerve cells typically having a high electroporation threshold. Depending on various parameters and conditions, the lower limit may also be set to higher values of for example 5 kV, 6 kV or 8 kV. Generally, the maximum voltage should not exceed a predefined upper limit of for example 20 kV, 15 kV, 12 kV or 10 kV in order to prevent negative effects such as arcing.

According to an embodiment, the PFA pulses are applied such as to generate an electric field of at least 10 kV/cm between the two electrodes. In other words, the voltage applied between the two electrodes may be set such that the electric field generated between the electrodes exceeds a predefined lower limit of 10 kV/cm. Particularly, such electric fields may be in a range of 11 - 15 kV/cm. Therein, the maximum electric field generated between the electrodes upon applying a specific voltage may depend on various parameters such as, first of all, a distance between the two electrodes but also other parameters such as a geometry of the electrodes, material characteristics of environmental materials such as electrical characteristics of blood and tissue adjacent to the electrodes, etc.

According to an embodiment, the PFA pulses are applied with pulse widths being between one nanoseconds (1 ns) and ten microseconds (10 µs). Applying the PFA pulses with such short pulse widths may improve an efficacy of the induced electroporation. Generally, the pulse width should be longer than a predefined lower limit of 1 ns or alternatively 5 ns, 10 ns, 20 ns, 50 ns, in order to induce sufficient electroporation in the nerves to be denervated.

However, the pulse width should be shorter than a predefined upper limit of 10 µs or alternatively 5 µs or 1 µs in order to prevent or limit negative effects such as electroporation in neighbouring cells not to be denervated or arcing between neighboring electrodes of different polarity. The pulse width is the duration during which the electric voltages is generated at the electrodes.

According to an embodiment, the PFA pulses are applied with interphase intervals being between hundred nanoseconds (100 ns) and ten microseconds (10 µs). Applying the PFA pulses with the interphase intervals being in such a range may improve an efficacy of the induced electroporation. The interphase interval may be a duration between successive phases in the PFA pulse.

According to an embodiment, the PFA pulses are applied with interpulse intervals being between ten microseconds (10 µs) and ten milliseconds (10 ms). Applying the PFA pulses with the interpulse intervals being in such a range may improve an efficacy of the induced electroporation. The interpulse interval may be a duration between successive pulses in sequence of PFA pulses.

According to an embodiment, the PFA pulses are applied in pulse trains, each pulse train including a plurality of PFA pulses. Therein, an interval between subsequent pulse trains is between ten milliseconds (10 ms) and two seconds (2 s). Alternatively or additionally, a number of pulse trains is between 5 and 200, preferably 10 and 100. In other words, a plurality of PFA pulses may be grouped in a pulse train and several of such pulse trains may be generated in a sequence. Therein interpulse intervals between pulses in a same pulse train are generally shorter then intervals between subsequent pulse trains, i.e. there is a substantial time gap between subsequent pulse trains. Setting the characteristics of the pulse trains to the indicated intervals may improve the efficacy of the induced electroporation.

In an embodiment a sequence of pulse trains could be applied as follows: a set of 5 to 20 pulse trains, followed by a pause of 10 seconds to 30 seconds, followed by another set of 5 to 50 pulse trains. Such a sequence may be repeated 2 to 20 times, preferably 5 to 10 times.

According to an embodiment, the PFA pulses are applied as biphasic pulses. Biphasic pulses comprise a positive and a negative pulse section. Generally, the pulse width is defined as the width of one of these sections, i.e. the width of the positive section or of the negative section. For example, in a biphasic pulse, a positive voltage amplitude may be applied in a first pulse section whereas a negative voltage amplitude may be applied in a second pulse section. Therein, the negative voltage amplitude may or may not be the inverse of the positive voltage amplitude. Applying biphasic PFA pulses may improve electroporation efficacy compared to monophasic pulses.

According to an embodiment, the PFA pulses are applied as charge-balanced pulses. Charge-balanced pulses may be biphasic pulses in which the positive and the negative section are designed to balance out each other. In other words, electric charges supplied in a first pulse section, a first pulse or a first sequence of pulses are subsequently compensated by opposite charges supplied in a second pulse section, a second pulse or a second sequence of pulses. Accordingly, a net charge delivered to cells or tissue may be as close to zero microcoulomb (0 µC) as possible. Charge-balancing the pulses may be beneficial in that chances of bubbling (gas formation induced by the electrolysis), arcing and/or undesired tissues stimulation may be minimized.

According to an embodiment, the method further comprises injecting an irrigation fluid in a volume in the renal vessel adjacent to at least one of the electrodes, the irrigation fluid being configured for reducing a local saline concentration in the volume.

In other words, a fluid, particularly the liquid, may be irrigated at a location at or close to the electrodes of the catheter. The irrigation fluid may therefore locally dilute for example blood flowing through the renal vessel adjacent to the electrodes. Generally, such blood has a significant saline concentration. Such saline concentration correlates with an ion concentration, thereby also influencing an electrical conductivity of the blood. Accordingly, by locally diluting the blood and reducing the saline concentration, a risk of arcing may be reduced during PFA pulse application.

The irrigation fluid may have a saline concentration of for example less than 0.4 vol-%, preferably less than 0.2 vol-% or even less than 0.1 vol-%. For example, the irrigation fluid may comprise water or may be water, preferably distilled water. A flow of irrigation fluid may be suitably set such as to achieve and maintain an intended saline concentration during PFA pulse delivery. Particularly, the fluid flow may be adapted such that, although the blood flow may continuously transfer the diluted blood away from the electrodes, replacing blood flowing to the volume around the electrodes is immediately diluted by injecting further irrigation fluid as long as further PFA pulses are to be generated.

For injecting such irrigation fluid, according to an embodiment, the catheter may comprise an irrigation fluid lumen for discharging the irrigation fluid at an opening into the volume adjacent to at least one of the electrodes. The irrigation fluid lumen may extend along the catheter. For example, the irrigation fluid lumen may establish a fluid connection between an irrigation fluid reservoir at a proximal end of the catheter and a discharge opening at the distal end of the catheter at or close to the electrodes. The irrigation fluid lumen may be connected to individual irrigation openings at the individual electrodes, in between the electrodes and/or proximal and distal to the most proximal and most distal electrode, respectively. Alternatively, the irrigation fluid lumen may extend between the catheter and an additional guiding catheter ending close to the distal end of the catheter carrying the electrodes in order to provide the irrigation fluid.

According to an embodiment, the method further comprises applying an electric pacing pulse to the vascular wall of the renal vessel at a position adjacent to nerves of the patient's sympathetic nervous system and detecting a response signal indicating a cardiac response of the patient's heart in reaction to the pacing pulse, in particular a change in blood pressure is used as response signal.

In other words, during the procedure of renal denervation, nerves or more generally cells at or adjacent to the vascular wall of the renal vessel may be specifically stimulated by applying an electric pacing pulse. The electric stimulation may reach the nerves of the patient's sympathetic nervous system and may induce a reaction therein. Such reaction may then induce another action which may finally result in provoking changes in a cardiac behaviour of the patient's heart. For example, pacing the sympathetic nervous system may generally result in increased blood pressure and/or heart rate. Such response in the cardiac activity of the patient's heart in reaction to the pacing pulse may be detected and may provide a suitable indication of a success of the renal denervation procedure

For example, according to a specific embodiment, the method may comprise
(i) applying a first electric pacing pulse to the vascular wall of the renal vessel at a position adjacent to nerves of the patient's sympathetic nervous system before applying the PFA pulses and detecting a first response signal indicating a cardiac response of the patients heart in reaction to the first pacing pulse,
(ii) applying a second electric pacing pulse to the vascular wall of the renal vessel at the position adjacent to nerves of the patient's sympathetic nervous system after applying the PFA pulses and detecting a second response signal indicating a cardiac response of the patients heart in reaction to the second pacing pulse, and
(iii) generating a denervation success signal indicating a degree of renal denervation resulting from the application of the PFA pulses, the denervation success signal being generated upon comparison of the first and second response signals.

Expressed differently, first and second electric pacing pulses may be applied before and after, respectively, applying the PFA pulses. For each of the pacing pulses, first and second response signals, respectively, may then be detected. Accordingly, these response signals indicate, on the one hand, the cardiac response to the first pacing pulse before the application of the PFA pulses and, on the other hand, the cardiac response to the second pacing pulse after the application of the PFA pulses. Generally, when the renal denervation procedure was successful, applying the second pacing pulse to the nerves of the sympathetic nervous system induces a lower or no reaction in these nerves and accordingly a lower or no cardiac response. Accordingly, by comparing the first and second response signals, it may be determined whether or not the renal denervation procedure was successful and a denervation success signal indicating a degree of renal denervation may be generated.

Particularly, according to an embodiment, the pacing pulse is applied such as to temporarily induce an electric pacing current to the nerves of the patient's sympathetic nervous system.

Therein, the electric pacing current exceeds 1 mA, preferably exceeds 5 mA or even 10 mA. Alternatively or additionally, the electric pacing current is induced during at least 1 ms or preferably at least 5 ms or even at least 10 ms.

In other words, an electric voltage may be applied between a pacing electrode and another electrode, the electric voltage being set such that an electric pacing current is induced temporarily with 1 mA or more. Such significant pacing current does generally not only reach the wall of the renal vessel but may also spread further beyond this wall and may then stimulate the nerves in the sympathetic nervous system behind this vascular wall. Such stimulation may be strong enough and/or may be maintained for a sufficient period of time such as to induce a substantial nerve reaction finally resulting in a detectable cardiac response to be represented by the response signal.

According to an embodiment, the response signal correlates to the patient's mean blood pressure, a temporal progress of the patient's blood pressure and/or the patient's heart rate.

In other words, in non-denervated nerves of the sympathetic nervous system, a pacing of such nerves generally induces a cardiac reaction in that a mean blood pressure temporarily increases by for example up to several mmHg or even more. Additionally or alternatively, the temporal progress of the blood pressure may be modified due to the pacing. Such modification may be detected for example by continuously monitoring the blood pressure or by analysing changes in a diastolic and/or a systolic blood pressure value. Furthermore, the heart rate may change due to the pacing stimulation of the sympathetic nervous system and may for example increased by up to several beats per minute. All these cardiac responses are generally at least partially suppressed upon the sympathetic nervous system having been successfully deactivated during renal denervation. Accordingly, by detecting or measuring parameters correlating with the blood pressure or heart rate and comparing values of these parameters acquired before and after the PFA pulse application, a response signal may be generated providing a valuable indication of the success of the renal denervation.

According to an embodiment, in order to enable the pacing pulse provision, the catheter may comprise at least one, preferably at least two pacing electrodes and may be connectable to a pacing pulse generator, the pacing electrode(s) being arranged at the distal end of the catheter and wherein the catheter is connectable to the pacing pulse generator being configured for generating an electric pacing pulse and applying the pacing pulse via the pacing electrode to nerves of the patient's sympathetic nervous system. The pacing electrode(s) may be an electrode solely used for pacing or an electrode used for applying the PFA pulses as well.

According to an embodiment, the electrodes are arranged in direct mechanical contact to an inner circumferential surface of the renal vessel.

In other words, for applying the PFA pulses, the electrodes are preferably positioned at the inner wall of the renal vessel and more preferably slightly pressed against the inner wall of the renal vessel. With such mechanical contact, the electric voltage may be applied directly to tissue of the vessel wall instead of being exclusively applied to blood within the vessel. Accordingly, the electric field may effectively affect nerve cells behind the vessel wall.

According to an embodiment, the PFA pulses are applied at various positions along a circumferential surface of the renal vessel such as to form an open-loop geometry.

Expressed differently, multiple electrodes may be arranged along the circumferential surface of the renal vessel at various positions. Therein, the positions are preferably not arranged in a closed-loop geometry, i.e. along a closed-loop circular line enclosing the inner volume of the vessel. Instead, the electrode positions are preferably arranged along a line which surrounds the inner volume of the vessel but which is an open-loop. In other words, the multiple electrodes are arranged at different positions with respect to the overall catheter axis. Various PFA pulses are then applied between two of the electrodes arranged in such configuration such that nerve cells are denervated along a line between these electrodes. Accordingly, after having applied PFA pulses to various pairs of electrodes, a line of denervated nerve cells may extend along a line following the open-loop geometry of the electrode arrangement. It has been observed that renal denervation applied in such open-loop geometry may prevent difficulties such as an occurrence of spasms in the renal artery upon applying the renal denervation which otherwise may occur when renal denervation is for example applied along a circular closed-loop geometry.

For example, according to an embodiment, the PFA pulses are applied at various positions along a helical line extending along a circumferential surface of the renal vessel. Such a helical line forms a preferred open-loop geometry for the resulting line of renal denervation.

According to an embodiment, the catheter comprises an expandable carrier structure at the distal end of the catheter, the expandable carrier structure carrying the plurality of electrodes. Accordingly, the electrodes used for renal denervation are arranged on the expandable carrier structure at a distal end of a catheter.

The expandable carrier structure may be configured such that it may be deformed from a retracted configuration to an expanded configuration. In the retracted configuration, the electrodes may be arranged such that most or all of the electrodes remain distant from the renal vessel wall upon the distal end of the catheter being inserted in the renal vessel. In the expanded configuration, the electrodes may be arranged such that most or all of the electrodes are distant from a center of the renal vessel and/or are arranged in mechanical contact with the renal vessel wall upon the distal end of the catheter being inserted in the renal vessel.

Having such expandable carrier structure, the catheter may first be inserted and guided through the vessel system of the patient with the carrier structure being in its retracted configuration until reaching an intended location in the renal vessel adjacent to the sympathetic nervous system. Having reached such location, the carrier structure may be deformed into its expanded configuration such as to dispose the electrodes at or close to the renal vessel wall.

For example, according to an embodiment, the expandable carrier structure is a helical carrier structure carrying the electrodes at various locations in a helical pattern along the helical carrier structure. In other words, the expandable carrier structure may be of a helix-type. Such expandable carrier structure may be deformed from a stretched, substantially linear configuration to a curved configuration in which it extends along a helical line. For example, the expandable carrier structure may be formed by a wire which is preconfigured into a helical shape. Such wire may be enclosed in a lumen of the catheter such as to be maintained in the linear configuration. In order to expand the carrier structure, the wire may be removed from the lumen such as to be deformed into its preconfigured helical shape.

As an alternative, according to an embodiment, the expandable carrier structure is a basket carrier structure comprising at least three splines carrying the electrodes at various locations in a helical pattern. The expandable carrier structure of such basket-type may comprise at least three splines provided at the end portion of the catheter. The splines may also be referred to as spines. The splines may be formed e.g. with wires having a preconfigured geometry. The splines may extend parallel to each other. In a retracted configuration, the splines may be arranged in close proximity to each other. For example, the splines may be held within a lumen of the catheter. In an expanded configuration, the splines may extend parallel and at a distance from each other such as to form a basket. For deforming into such basket configuration, the splines may be removed from the lumen of the catheter. The electrodes are arranged on the splines. Preferably, the electrodes are arranged at longitudinal positions along the splines, such longitudinal positions being different for each of the splines. In other words, the electrodes are preferably not arranged at each of the splines at the same longitudinal position along the extension direction of the catheter. Instead, the electrodes are arranged such as to be staggered in a helical pattern.

According to an embodiment, at least one of various conditions may apply with regard to the helical carrier pattern:
(i) The helical carrier pattern may have at least two turns, preferably at least four turns. In other words, the electrodes may be arranged along a helix line extending along at least 720°, preferably extending along 1440° or more.
(ii) The helical carrier pattern may have a length of at least 3 cm, preferably at least 5 cm. In other words, the length of the helical carrier pattern in a direction parallel to a centreline thereof may be 3 cm or more. Particularly, each 360° turn of the helical pattern may have a length of between 1 cm and 2 cm, e.g. 1.5 cm.
(iii) The helical carrier pattern may have a fit of at least 2.5, preferably at least 3.5, e.g. 3.6 electrodes. This may mean that approximately two pairs of electrodes are provided per turn of the helical carrier pattern.
(iv) The helical carrier pattern may include at least 8 electrodes, preferably at least 14 electrodes. Expressed differently, four or more, particularly seven, pairs of electrodes may be provided along the helical carrier pattern.

Providing the electrodes along a helical carrier pattern having at least one of such characteristics may enable generating an advantageous renal denervation with regards to its efficacy and/or other medical effects.

According to another embodiment, the catheter may comprise a linear carrier structure at the distal end of the catheter, the linear carrier structure carrying the plurality of electrodes. In other words, the distal end of the catheter may be provided as a simple, non-expandable linear carrier structure. Such linear carrier structure may be mechanically less complex and/or easier to handle than an expandable carrier structure.

Furthermore, according to an embodiment, the catheter may comprise a steering mechanism configured for steering the distal portion of a catheter. Using such steering mechanism, the distal portion of the catheter may therefore be guided into an intended direction and/or deformed into an intended geometry. For example, the distal portion of the catheter may include one, two or more longitudinal steering guides such as steering wires or cables for moving and/or bending the catheter's distal portion.

Accordingly, according to an embodiment of the renal denervation method, the electrodes are arranged at the steerable distal portion of the catheter and the electrodes are positioned at the vascular wall of the renal vessel by suitably steering the distal portion of the catheter. For example, the steerable distal portion may be specifically arranged such that one or some of the electrodes are arranged at one surface portion of the renal vessel whereas another one or some other electrodes are arranged at an opposite surface portion of the renal vessel. During the renal denervation procedure, the steerable distal portion and the electrodes disposed thereon may be displaced one or several times such as to arrange the electrodes at various locations along for example a helical pattern or another open-loop pattern.

According to an embodiment, at least one of the following conditions may apply:
(i) An even number of electrodes is arranged at the distal end of the catheter. For example, 4, 6, 8, 10, 12, 14 or more electrodes may be provided.
(ii) The electrodes have a length of 0.5 - 5 mm, preferably 1 - 3 mm.
(iii) The electrodes are distributed along a length of 2 - 10 cm, preferably 4 - 8 cm,
(iv) A distance between adjacent electrodes is 0.3 - 5 mm, preferably 0.5 - 3 mm,
(v) An outer diameter of the distal end of the catheter is 3 - 9 mm, preferably 4 - 6 mm. For example, the catheter comprising the distal end could be 4 - 8 F (4 - 8 French) in the case of a linear distal end or 5 - 6 F in the case of a helical or basket-type distal end.

It shall be noted that possible features and advantages of embodiments of the invention are described herein partly with respect to a method for renal denervation and partly with respect to a catheter specifically configured for such renal denervation. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.

The figures are only schematic and not to scale. Same reference signs refer to same or similar features.
- Fig. 1: shows a catheter for renal denervation according to an embodiment of the present invention.
- Figs. 2a,b: show PFA pulses supplied in a method for renal denervation according to an embodiment of the present invention.
- Fig. 3: shows a perspective view of a helix-type distal portion of a catheter for renal denervation according to an embodiment of the present invention.
- Fig. 4: shows a perspective view of a basket-type distal portion of a catheter for renal denervation according to an embodiment of the present invention.
- Fig. 5: shows an end view of the basket-type distal end of Fig. 4.

The figures are only schematic and not to scale. Same reference signs indicate same or similar features throughout the figures.

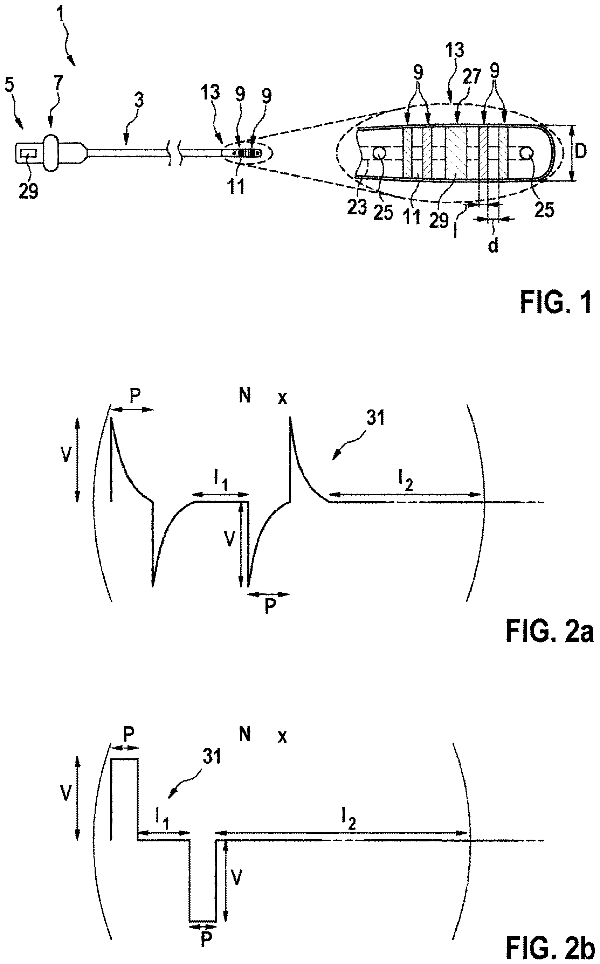

Figure 1 shows a catheter 1 for renal denervation. The catheter 1 comprises an elongate shaft 3. At a proximal end, the catheter 1 comprises a handle 5 with a steering mechanism 7. At a distal portion 13 of the catheter 1, two or more electrodes 9 are provided on a linear, cylindrical carrier structure 11. The electrodes 9 are configured for applying electric PFA pulses to tissue or fluid being in electric contact with the electrodes 9. The electrodes 9 are ring electrodes extending along a circumference of the carrier structure 11. While only four electrodes 9 (i.e. two pairs of electrodes) are exemplarily shown in the figure, a larger number, for example up to twelve electrodes, may be provided at the distal end of the catheter 1. Each electrode 9 may have a length l, measured in the longitudinal direction of the catheter 1, of 1 mm to 3 mm. Neighbouring electrodes 9 may be arranged at a distance d of between 0.5 mm and 3 mm. A diameter D of the catheter's distal portion 13 or of the carrier structure 11 may be between 3 mm and 9 mm, i.e. the catheter 1 may comprise a distal end with 4 - 6 F.

The steering mechanism 7 is configured for steering the distal portion 13 of the catheter 1 such as to deform or bend the distal portion 13 into a configuration in which the electrodes 9 are arranged at intended locations. For example, the electrodes 9 may be arranged at or close to a vascular wall into which the catheter's distal portion 13 has been introduced. For such purpose, the steering mechanism 7 may include steering wires extending from the handle 5 through the elongate shaft 3 up to anchoring fixations at the distal portion 13.

The electrodes 9 are electrically connected to a PFA pulse generator (not shown) via electrical connections 29. For such purpose, electric wires may extend through the shaft 3 and may be connected to each of the electrodes 9. For example, the catheter 1 may comprise at least two lumens for individual connectors connected to the electrodes 9, whereby wires or generally conductors connected to electrodes 9 of a same polarity may be placed within the same lumen. The PFA pulse generator may be electrically connected to an interface 29 provided at the handle 5. The PFA pulse generator is configured for generating high voltage pulses of for example at least 4 kV and a pulse length in a range of several nanoseconds to ten microseconds. The pulses are biphasic and charge-balanced.

Without limitations, as an example, Fig. 2a shows a waveform of a biphasic exponentially decaying voltage pulses 31 suitable for PFA treatment. Over the entire waveform complex, the exponential decays achieve a charge-balanced goal, needed to minimize chances of bubbling, arcing or undesired tissue stimulation. Such waveforms may be achieved by using high-voltage output stages which are AC-coupled to the ablation electrodes 9. The two biphasic pulses shown in Fig. 2a could be repeated N-times forming a pulse train. The biphasic pulses consist of a positive section PP and a negative section PN. As shown in Fig. 2a, a positive biphasic pulse is followed by an inverse negative biphasic pulse. An interphase delay I₁ or interphase interval is a time between an end of the negative section PN of the first biphasic pulse and a start of the positive section of the following pulse. A pulse width P corresponds to the length of the positive/negative section PP/PN. The next pulse starts after a interpulse delay I₂ or interpulse interval.

Similarly, Fig. 2b shows an example of suitable PFA waveforms which have rectangular shapes. The rectangular pulses as shown in Fig. 2b are characterized by the voltage peak V and the pulse width P. A positive rectangular pulse is followed by a negative rectangular pulse after the interphase delay I₁. The two pulses shown in Fig. 2b could be repeated N-times forming a pulse train. The next pulse starts after the interpulse delay I₂. These waveforms are also charge-balanced. Such charge-balanced rectangular waveforms may be achieved by using DC-coupled high-voltage output stages with reasonably accurate control of the positive and negative phase amplitude and duration. As a result, the net charge (current amplitude*pulse width) can be controlled to achieve net balancing.

For renal denervation as described herein, preferred parameters may be set. For example, the peak voltage V may be set to 4 kV or more, preferably between 4 kV and 15 kV or between 4 kV and 10 kV. The pulse width P may be set to 1 ns - 10 ms or 5 ns - 2 ms or 100 ns-1 ms. The interphase interval I₁ may be set to 1 ms - 10 ms. The interpulse interval I₂ may be set to 10 µs - 10 ms. Each pulse train may comprise 10- 100 pulses. An interval between pulse trains may be 10 ms - 2 s.

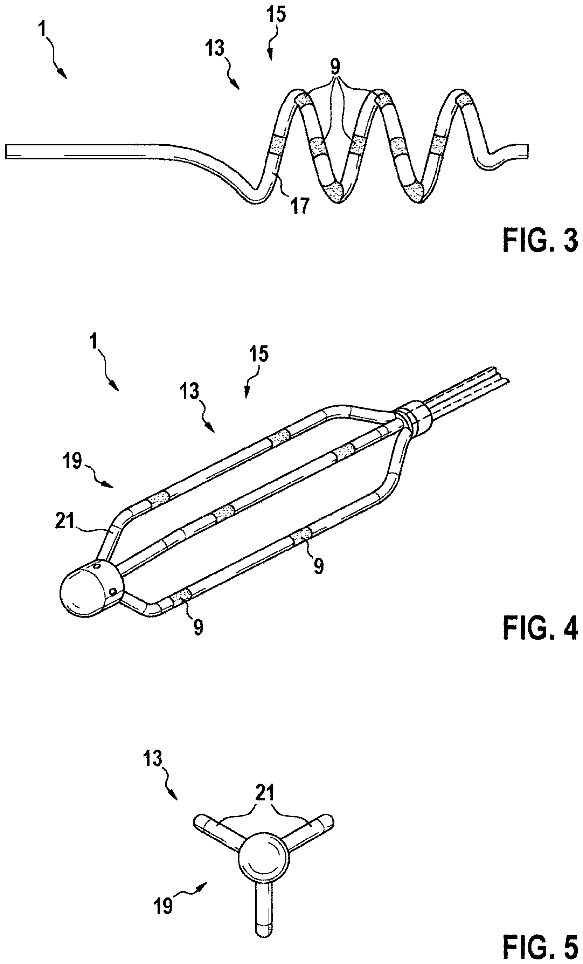

Fig. 3 shows a perspective view of a distal portion 13 of the catheter 1 with an expandable carrier structure 15 implemented with a helical carrier structure 17. The helical carrier structure 17 carries several electrodes 9 at various locations in a helical pattern. While being visualised in a simplified manner, the actual helical pattern may comprise for example a helix with 4 turns distributed over a length of 6 cm. The turns generally have a constant diameter. An outer diameter of the turns of the helical carrier structure roughly corresponds to an inner diameter of a typical renal artery. The outer diameter of the turns of the helical carrier structure may be between 2 mm and 15 mm, in particular between 3 mm and 10 mm, in particular between 4 mm and 7 mm. A height of each turn (e.g. the distance between two adjacent loops) may be approximately 1.5 cm. A fit may be approximately 3.6 electrodes (i.e. approximately 2 pairs per turn). 14 electrodes, i.e. 7 pairs, may be provided on the helical carrier structure 17.

The distal portion 13 is preconfigured into the helical shape and has a central lumen to deliver over the wire. Once inside the renal artery, the wire is removed to allow the helix to expand.

In an alternative approach, the helical carrier structure 17 is initially loosely wound. After it is deployed inside the renal artery, a distal end is tightened to achieve better contact with the arterial wall.

Fig. 4 and Fig. 5 show a perspective view and an end view of a distal portion 13 of the catheter 1 with an expandable carrier structure 15 implemented with a basket carrier structure 19. The basket carrier structure 19 comprises three splines 21. In a major portion thereof, the splines 21 extend parallel to each other and spaced apart from each other. A lateral distance between the splines 21 is set such that each of the splines 21 may come into lateral contact of a vascular wall of a renal vessel. Each spline 21 carries several electrodes 9. Overall, the electrodes 9 are arranged at various locations staggered in a helical pattern.

The catheter 1 may then be inserted into the renal artery while the distal portion 13 is inside a renal introducer sheath with the baskets carrier structure 19 being collapsed. After it is inside a target area within the renal artery, the basket carrier structure 19 is expanded or deployed.

In an alternative approach, the basket splines 21 are comprised of an LCP flexible substrate. The electrodes 9 are staggered again in a helical configuration.

As shown schematically in Fig. 1, the catheter 1 further comprises an irrigation fluid lumen 23. This irrigation fluid lumen 23 connects openings 25 provided at the distal portion 13 in close neighbourhood to the electrodes 9 with an irrigation fluid reservoir (not shown) for example provided at or connected with the handle 5.

Accordingly, irrigation fluid such as for example distilled water may be injected at a treatment site adjacent to the electrodes 9. The irrigation fluid may help reducing a local concentration of saline in blood at the treatment site. Accordingly, a risk of arcing may be reduced. The irrigation lumen 23 may be connected to individual irrigation openings 25 at the individual electrodes 9, in between the electrodes 9 or proximal and distal to the most proximal and most distal electrode 9, respectively.

In an alternative embodiment, a lumen 23 between the catheter 1 and an additional guiding catheter (not shown) ending close to the distal end of the catheter 1 carrying the electrodes 9 could be used to provide the irrigation fluid.

As also shown schematically in Fig. 1, the catheter 1 may comprise one, two or more pacing electrodes 27. The pacing electrodes 27 may be arranged on the carrier structure 11. Preferably, the pacing electrodes 27 are provided in close proximity to the electrodes 9 for providing the PFA pulses. For example, a pacing electrode 27 may be arranged between PFA electrodes 9 of a pair of such electrodes 9 or between two pairs of such electrodes 9. The pacing electrodes 27 may be electrically connected to a pacing voltage generator (not shown) connected to the catheter 1. Accordingly, electric pacing pulses may be applied via the pacing electrodes 27 to a vascular wall of the renal vessel at a position adjacent to nerves of the patient's sympathetic nervous system (SNS), whereby either the electrodes 9 or an electrode provided as a patch at the skin of the patient may serve as counter electrode for pacing electrodes 27.

Pacing the sympathetic nerves through the renal artery wall may offer an indication of acute technical success of a procedure for renal denervation. By stimulating the SNS prior to the ablation procedure, a cardiac response such as a blood pressure increase and/or a heart rate change baseline may be established. After having successfully completed the renal denervation procedure, a pacing of the SNS will induce little or no change in the observed blood pressure and/or heart rate.

With the approach described herein and particularly with the catheter 1 described herein, a method for renal denervation may be established in which nerves of the patient's SNS adjacent to the renal artery may be denervated. For such purpose, the distal portion 13 of the catheter 1 may be arranged with its electrodes 9 being adjacent or in direct contact with the vascular wall of the renal vessel at a position adjacent to nerves of the SNS. PFA pulses are then applied to the electrodes 9 such as to induce electroporation in nerve cells of the SNS. Simultaneously, irrigation fluid is injected to a volume in the renal vessel adjacent to the electrodes 9 for reducing a local saline concentration, thereby reducing a risk of arcing. Before and after the PFA pulses are applied, a pacing pulse is applied to the SNS and a response signal indicating a cardiac response of the patient's heart in reaction to the pacing pulse is detected in each case. By comparison of the first and second response signals, a denervation success signal indicating a degree of renal denervation resulting from the application of the PFA pulses is generated.

Overall, recent research in cardiac ablations indicate that fibrotic healing of cells which are pulse field ablated generally occurs faster and with less inflammation processes compared to RF ablated tissue. In addition, overheating with RF ablation is a known risk that could lead to severe structural destruction, while PFA is known to maintain structural integrity of an extracellular matrix.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

In view of all the foregoing disclosure, the present invention also provides for the following consecutively numbered embodiments:
1. A method for renal denervation, the method comprising:
   applying electric PFA pulses (31) to a vascular wall of a renal vessel at a position adjacent to nerves of a patient's sympathetic nervous system,
   wherein the PFA pulses (31) are applied via at least two electrodes (9) of a catheter (1) arranged within the renal vessel,
   wherein the PFA pulses (31) are configured such as to induce electroporation in nerve cells of the patient's sympathetic nervous system.
2. The method according to embodiment 1,
   wherein the PFA pulses (31) are applied by generating a voltage (V) of at least 4 kV between the two electrodes.
3. The method according to any of the embodiments,
   wherein the PFA pulses (31) are applied such as to generate an electric field of at least 10 kV/cm between the two electrodes.
4. The method according to any of the previous embodiments,
   wherein the PFA pulses (31) are applied with pulse widths (P) being between 1 ns and 10 µs.
5. The method according to any of the previous embodiments,
   wherein the PFA pulses (31) are applied with interphase intervals (Ii) being between 100ns and 10 µs.
6. The method according to any of the previous embodiments,
   wherein the PFA pulses (31) are applied with interpulse intervals (I₂) being between 10 µs and 10 ms.
7. The method according to any of the previous embodiments,
   wherein the PFA pulses (31) are applied in pulse trains, each pulse train including a plurality of PFA pulses, wherein at least one of the following condition applies:
   - an interval between subsequent pulse trains is between 10 ms and 2 s,
   - a number of pulse trains is between 5 and 200, preferably 10 and 100.
8. The method according to any of the previous embodiments,
   wherein the PFA pulses (31) are applied as biphasic pulses.
9. The method according to any of the previous embodiments,
   wherein the PFA pulses (31) are applied as charge-balanced pulses.
10. The method according to any of the previous embodiments, further comprising injecting an irrigation fluid in a volume in the renal vessel adjacent to at least one of the electrodes (9), the irrigation fluid being configured for reducing a local saline concentration in the volume.
11. The method according to any of the previous embodiments, further comprising applying an electric pacing pulse to the vascular wall of the renal vessel at a position adjacent to nerves of the patient's sympathetic nervous system and detecting a response signal indicating a cardiac response of the patient's heart in reaction to the pacing pulse.
12. The method according to any of the previous embodiments, further comprising applying a first electric pacing pulse to the vascular wall of the renal vessel at a position adjacent to nerves of the patient's sympathetic nervous system before applying the PFA pulses and detecting a first response signal indicating a cardiac response of the patient's heart in reaction to the first pacing pulse,
   applying a second electric pacing pulse to the vascular wall of the renal vessel at the position adjacent to nerves of the patient's sympathetic nervous system after applying the PFA pulses and detecting a second response signal indicating a cardiac response of the patient's heart in reaction to the second pacing pulse, and
   generating a denervation success signal indicating a degree of renal denervation resulting from the application of the PFA pulses, the denervation success signal being generated upon comparison of the first and second response signals.
13. The method according to any of the embodiments 11 and 12,
   wherein the pacing pulse is applied such as to temporarily induce an electric pacing current to the nerves of the patient's sympathetic nervous system, wherein at least one of the following conditions applies:
   - the electric pacing current exceeds 1 mA;
   - the electric pacing current is induced during at least 1 ms.
14. The method according to any of the embodiments 11 to 13,
   wherein the response signal correlates to at least one of the patient's mean blood pressure, a temporal progress of the patient's blood pressure and the patient's heart rate.
15. The method according to any of the previous embodiments,
   wherein the electrodes (9) are arranged in direct mechanical contact to an inner circumferential surface of the renal vessel.
16. The method according to any of the previous embodiments,
   wherein the PFA pulses (31) are applied at various positions along a circumferential surface of the renal vessel such as to form an open-loop geometry.
17. The method according to any of the previous embodiments,
   wherein the PFA pulses are applied at various positions along a helical line extending along a circumferential surface of the renal vessel.
18. The method according to any of the previous embodiments,
   wherein the electrodes are arranged on an expandable carrier structure (15) at a distal portion (13) of a catheter (1).
19. The method according to any of the previous embodiments,
   wherein the electrodes (9) are arranged on one of
   a helical carrier structure (17) carrying the electrodes (9) at various locations along the helical carrier structure (17), and
   a basket carrier structure (19) comprising at least three splines (21) carrying the electrodes (9) at various locations in a helical pattern.
20. The method according to any of the previous embodiments,
   wherein the electrodes (9) are arranged at a steerable distal portion (13) of a catheter (1) and wherein the electrodes (9) are positioned at the vascular wall of the renal vessel by suitably steering the distal portion (13) of the catheter (1).
21. A catheter for renal denervation, comprising:
   a plurality of electrodes (9) arranged at a distal portion (13) of the catheter (1),
   wherein the catheter is configured for connecting to a PFA pulse generator configured for generating electric PFA pulses,
   wherein the catheter (1) is configured for implementing the method for renal denervation according to one of the previous claims.
22. The catheter according to embodiment 21, further comprising an irrigation fluid lumen (23) for discharging an irrigation fluid at an opening (25) into a volume adjacent to at least one of the electrodes (9).
23. The catheter according to one of embodiments 21 and 22, further comprising a pacing electrode (27) arranged at the distal portion (13) of the catheter (1), and wherein the catheter is connectable to a pacing pulse generator configured for generating an electric pacing pulse and applying the pacing pulse via the pacing electrode (27) to nerves of the patient's sympathetic nervous system.
24. The catheter according to one of embodiments 21 to 23, further comprising an expandable carrier structure (15) at the distal portion (13) of the catheter (1), the expandable carrier structure (15) carrying the plurality of electrodes (9).
25. The catheter according to embodiment 24,
   wherein the expandable carrier structure (15) is a helical carrier structure (17) carrying the electrodes (9) at various locations in a helical pattern along the helical carrier structure (17).
26. The catheter according to embodiment 24,
   wherein the expandable carrier structure (15) is a basket carrier structure (19) comprising at least three splines (21) carrying the electrodes (9) at various locations in a helical pattern.
27. The catheter according to one of embodiments 25 and 26,
   wherein at least one of the following conditions applies:
   - the helical carrier pattern has at least two turns, preferably at least four turns,
   - the helical carrier pattern has a length of at least 3 cm, preferably at least 5 cm,
   - the helical carrier pattern has a fit of at least 2.5, preferably at least 3.5,
   - the helical carrier pattern includes at least 8 electrodes, preferably at least 14 electrodes.
28. The catheter according to one of embodiments 21 to 23, further comprising a linear carrier structure (11) at the distal portion (13) of the catheter (1), the linear carrier structure (11) carrying the plurality of electrodes (9).
29. The catheter according to one of embodiments 21 to 28, further comprising a steering mechanism (7) configured for steering the distal portion (13) of a catheter (1).
30. The catheter according to one of embodiments 21 to 29,
   wherein at least one of the following conditions applies:
   - an even number of electrodes (9) is arranged at the distal portion of the catheter (1),
   - the electrodes (9) have a length (l) of 0.5 - 5 mm, preferably 1-3 mm,
   - the electrodes are distributed along a length of 2 - 10 cm, preferably 4 - 8 cm,
   - a distance (d) between adjacent electrodes is 0.3 - 5 mm, preferably 0.5 - 3 mm,
   - an outer diameter (D) of the distal end of the catheter is 3 - 9 mm, preferably 4 - 6 mm
31. A system comprising a catheter according to any of the embodiments 21 to 30 and configured for implementing the method according to any of the embodiments 1 to 20.
32. The system according to embodiment 31 comprising a pulse generator connected to the proximal end, in particular to the handle, of the catheter.
33. The system according to embodiment 32, wherein the pulse generator is configured to generate pulses according to embodiments 2 to 13.

### List of reference signs:

- 1: catheter
- 3: shaft
- 5: handle
- 7: steering mechanism
- 9: electrode
- 11: carrier structure
- 13: distal portion
- 15: expandable carrier structure
- 17: helical carrier structure
- 19: basket carrier structure
- 21: spline
- 23: irrigation fluid lumen
- 25: opening
- 27: pacing electrodes
- 29: PFA pulse generator
- 31: PFA pulse
- 33: pacing pulse generator

## Claims

1. A method for renal denervation, the method comprising:
applying electric PFA pulses (31) to a vascular wall of a renal vessel at a position adjacent to nerves of a patient's sympathetic nervous system,
wherein the PFA pulses (31) are applied via at least two electrodes (9) of a catheter (1) arranged within the renal vessel,
wherein the PFA pulses (31) are configured such as to induce electroporation in nerve cells of the patient's sympathetic nervous system.

2. The method according to claim 1,
wherein the PFA pulses (31) are applied by generating a voltage (V) of at least 4 kV between the two electrodes.

3. The method according to any of the previous claims,
wherein the PFA pulses (31) are applied such as to generate an electric field of at least 10 kV/cm between the two electrodes.

4. The method according to any of the previous claims,
wherein the PFA pulses (31) are applied with pulse widths (P) being between 1 ns and 10 µs.

5. The method according to any of the previous claims,
wherein the PFA pulses (31) are applied with interphase intervals (Ii) being between 100 ns and 10 µs.

6. The method according to any of the previous claims,
wherein the PFA pulses (31) are applied with interpulse intervals (I₂) being between 10 µs and 10 ms.

7. The method according to any of the previous claims,
wherein the PFA pulses (31) are applied in pulse trains, each pulse train including a plurality of PFA pulses, wherein at least one of the following condition applies:
- an interval between subsequent pulse trains is between 10 ms and 2 s,
- a number of pulse trains is between 5 and 200, preferably 10 and 100.

8. The method according to any of the previous claims,
wherein the PFA pulses (31) are applied as biphasic pulses.

9. The method according to any of the previous claims,
wherein the PFA pulses (31) are applied as charge-balanced pulses.

10. A catheter for renal denervation, comprising:
a plurality of electrodes (9) arranged at a distal portion (13) of the catheter (1),
wherein the catheter is configured for connecting to a PFA pulse generator (29) configured for generating electric PFA pulses,
wherein the catheter (1) is configured for implementing the method for renal denervation according to one of the previous claims.

11. The catheter according to claim 10, further comprising
an irrigation fluid lumen (23) for discharging an irrigation fluid at an opening (25) into a volume adjacent to at least one of the electrodes (9).

12. The catheter according to one of claims 10 and 11, further comprising a pacing electrode (27) arranged at the distal portion (13) of the catheter (1), and wherein the catheter is connectable to a pacing pulse generator (33) configured for generating an electric pacing pulse and applying the pacing pulse via the pacing electrode (27) to nerves of the patient's sympathetic nervous system.

13. The catheter according to one of claims 10 to 13, further comprising an expandable carrier structure (15) at the distal portion (13) of the catheter (1), the expandable carrier structure (15) carrying the plurality of electrodes (9).

14. The catheter according to claim 13,
wherein the expandable carrier structure (15) is a helical carrier structure (17) carrying the electrodes (9) at various locations in a helical pattern along the helical carrier structure (17).

15. The catheter according to claim 14,
wherein the expandable carrier structure (15) is a basket carrier structure (19) comprising at least three splines (21) carrying the electrodes (9) at various locations in a helical pattern.
